# EUROPÄISCHE PATENTSCHRIFT

(11) **EP 3 339 831 B1**
(45) Veröffentlichungstag und Bekanntmachung des Hinweises auf die Patenterteilung: **07.09.2022**
(21) Anmeldenummer: 17209715.6
(22) Anmeldetag: 21.12.2017
(51) Int. Cl.: G01N 1/42, A61B 10/00

(54) **VORRICHTUNG FÜR DAS KÜHLEN SOWIE DAS EINFRIEREN UND KÜHLHALTEN VON BIOLOGISCHEN UND/ODER MEDIZINISCHEN PROBEN SOWIE VERFAHREN ZUM BETREIBEN DER VORRICHTUNG**
APPARATUS FOR COOLING AS WELL AS FREEZING AND MAINTAINING BIOLOGICAL AND/OR MEDICAL SAMPLES IN A COLD STATE, AND METHOD OF OPERATING THE APPARATUS
DISPOSITIF POUR LE REFROIDISSEMENT AINSI QUE LA CONGÉLATION ET LE MAINTIEN AU FROID D'ÉCHANTILLONS BIOLOGIQUES ET/OU MÉDICAUX ET PROCÉDÉ D'UTILISATION DU DISPOSITIF

(30) Priorität: 22.12.2016 DE 102016125444
(43) Veröffentlichungstag der Anmeldung: 27.06.2018
(73) Patentinhaber: BioKryo GmbH, 66117 Saarbrücken (DE)
(72) Erfinder: von Walcke-Wulffen, Vincent, 66119 Saarbrücken (DE); Schmidt, Tomm, 28865 Lilienthal (DE)
(74) Vertreter: Patentanwaltskanzlei Vièl & Wieske PartGmbB

(56) Entgegenhaltungen:
- EP-A2- 0 275 829
- DE-A1- 2 657 703
- FR-A1- 2 150 937

## Beschreibung

Die vorliegende Erfindung betrifft eine Vorrichtung für das Kühlen von biologischen und/oder medizinischen Proben, eine Vorrichtung für das Einfrieren und Kühlhalten von biologischen und/oder medizinischen Proben sowie ein Verfahren zum Betreiben der Vorrichtung.

Vorrichtungen zum Kühlen und Tiefgefrieren von lebenden biologischen Proben sind bekannt. Beispielsweise offenbart die EP 0 275 829 A2 eine Vorrichtung zum Tiefgefrieren von Zellen, bei der durch eine Kaskade aus mehreren Stufen thermisch in Serie geschalteter Peltierelemente ein gleichmäßiges Abkühlen der lebenden biologischen Proben erfolgt, ohne dass Zellschädigung durch zu schnelles oder zu langsames Abkühlen auftritt. Die DE 26 57 703 A1 offenbart für eine Vorrichtung und ein kryogenes Verfahren zum Gefrieren von in Biobehältern abgefüllten biologischen Substanzen, die zur Vermeidung von irreversiblen Zellschädigungen während des Gefriervorgangs nur mit niedrigen Gefriergeschwindigkeiten eingefroren werden dürfen, dass die Biobehälter zwischen Platten aus schlecht wärmeleitendem Material eingelegt werden.

Bioproben, die bereits im Operationssaal (OP) oder in der Ambulanz durch Probeentnahmen (z.B. Darmspiegelung mit Gewebeproben bzw. Flüssigbiopsien wie z.B. Blut, Sputum oder Liquor) oder im Rahmen einer Labortätigkeit oder im Rahmen von Feldversuchen gewonnen werden und schnell tiefgefroren werden, sind für die Auswahl der Behandlung in der klinischen Routineversorgung von Patienten sowie für molekulardiagnostische Anwendungen zur personalisierten oder stratifizierten Medizin von immenser Bedeutung.

Verschiedene exogene Faktoren beeinflussen hierbei die molekulare Komposition von humanem Gewebe vor, während und nach einer Probenentnahme. Zu diesen exogenen Faktoren gehört beispielsweise die Ischämie, eine Unterversorgung des Gewebes mit Blut, die innerhalb von Minuten zu einer Veränderung der Gen- und Proteinexpressionsmuster und zudem zu einer Veränderung des Phosphorylierungsstatus von Proteinen im Proben (Gewebeprobe oder Flüssigbiopsieprobe) führt. Eine Strategie zur Entnahme und zum Einfrieren von Proben ist deshalb, diese Faktoren zu minimieren, um die tatsächlich vorliegenden molekularen Charakteristika der Probe für weitere Analysen zu erhalten.

Ein somit sehr wichtiger Aspekt für molekulardiagnostische Anwendungen und damit die neuartige Behandlung ist daher die Probengewinnung und -aufarbeitung für die Analysen. Nur bei einer kontrollierten, definierten und schnell durchgeführten Probenverarbeitung ist es möglich, die biologischen Unterschiede zwischen Proben zu erhalten, die letztendlich zur Unterscheidung von Patienten auf molekularer Ebene und zur Entwicklung von zielgerichteten molekularen Therapien führen können.

Das Probenmaterial wird während der Probeentnahme zur Diagnosesicherung oder während der operativen Entfernung eines Tumors entnommen. Ein entscheidender Faktor ist, dass vor pathologischer Begutachtung das Gewebe bzw. die Flüssigbiopsie schnellstmöglich konserviert werden muss, um insbesondere die mRNA Moleküle der jeweilig zu untersuchenden Gene in ihrer gesamten Quantität und Qualität zu erhalten.

Durch quantitative Analyse der mRNA Moleküle kann bestimmt werden, ob ein Gen ungewöhnlich häufig abgelesen, also stark exprimiert wird, oder ob die Expression unterdrückt wird. Im Falle der Feststellung eines Tumors kann durch die Analyse ein Profil des Tumors erstellt werden.

Auch die Quantität und Qualität der Proteine in den Zellen muss erhalten bleiben, da diese weitere Hinweise für die Art der Erkrankung, beispielsweise Art des Krebses, und erfolgversprechende Therapien bzw. für das Therapieansprechen liefern können.

Nach neuen Erfahrungen weiß man zudem, dass es kleine RNA Moleküle gibt, die nicht aus funktionellen Genen (Genen, die den Bauplan für Proteine beinhalten) resultieren, sondern aus sogenannten nicht-kodierenden Regionen. Diese sog. smallRNAs oder ncRNAs bzw. IncRNAs haben ebenfalls Einfluss auf die Bildung von Proteinen, da sie regulierend wirken.

Daher ist es für subsequente Analysemethoden ebenfalls relevant, auch diese während der Konservierung zu erhalten.

Für alle oben genannten Moleküle ist es unbedingt notwendig, dass die Zeit der Ischämie (Gewebe ist vom Blutkreislauf abgeschnitten, die Sauerstoffzufuhr ist somit unterbrochen, das Gewebe beginnt abzusterben) möglichst kurzgehalten wird.

Eine zurzeit übliche Methode der Konservierung in Krankenhäusern und ambulanten Kliniken ist das Einlegen der Gewebestücke in Formalin, einer Formaldehyd-haltigen Lösung, mit der folgenden Einbettung in Paraffin oder einem anderen Wachs. So werden die Proben vorbereitet, um Tumorgewebeschnitte durchzuführen, die vom Pathologen für die genaue histologische Diagnose benötigt werden.

Obwohl auch aus diesen Schnitten Biomoleküle gewonnen werden können, kommt es jedoch in vielen Fällen zu einer Destabilisierung vor allem großer RNA Fragmente und sogar zu Veränderungen der RNA. Diese Veränderungen treten ungleichmäßig auf, das heißt, sie können in der Analyse nur bedingt mathematisch nachvollzogen und berücksichtigt werden.

Weiterhin führen die eingesetzten Fixiermittel, wie z.B. Formalin, grundsätzlich zu einer Kreuzvernetzung von Proteinen in Geweben, indem sie Methylenbrücken und Brücken per Schiff"sche Basen ausbilden. Diese Prozesse können die RNA schädigen und die antigenen Zielstrukturen verändern, indem sie diese maskieren oder die Bindestellen für Antikörper zerstören.

Somit führen die Formalinfixation-induzierten Veränderungen zu suboptimalen *in situ* Analysen von DNA, RNA und Proteinen und beeinflussen - u. a. auch durch Variationen in der Einwirkzeit - signifikant die Qualität sowie die Reproduzierbarkeit von Ergebnissen.

Vorteilhaft können diese Nachteile durch den Einsatz von tiefgefrorenen Geweben für anschließende Analysen überwunden werden. Untersuchungen, in welchen histopathologische Diagnosen von tiefgefrorenen Tumorgeweben (Gefrierschnitte) verschiedener Krebsarten evaluiert wurden, zeigen eine Sensitivität und Spezifität nahe 100%. Dies ergibt sich aus den folgenden Veröffentlichungen:
> Saina Attaran, Gentjan Jakaj, Metesh Acharya and Jon R. Anderson: "Are frozen sections of mediastinoscopy samples as effective as formal paraffin assessment of mediastinoscopy samples for a decision on a combined mediastinoscopy plus lobectomy?", Interactive Cardio Vascular and Thoracic Surgery 16 (2013) 872-874 Boriboonhirunsam, A.; Semboon, A.;" Accuracy of frozen sections in the diagnosis of malignant ovarian tumor" Journal of Obstetrics and Gynaecology Research Vol. 30 Issue 5 pages 394-399 October 2004

Diese Gefrierschnitte eignen sich auch hervorragend für den Einsatz in der Telepathologie. Durch den Einsatz digitaler Pathologieinstrumente verändert sich momentan die medizinische Praxis in Richtung Zentralisierung und Spezialisierung, so dass hier ein breites Anwendungsgebiet für tiefgefrorene Gewebe wächst. Dies gilt insbesondere dann, wenn diese Gewebe nach dem Tieffrieren auch in dem tiefgefrorenen Zustand transportierbar sind.

Aus den oben genannten Gründen ist die optimale Methode der Konservierung das sogenannte "Snap Freezing", ein schneller Einfrierprozess, der gewährleistet, dass die Biomoleküle unverändert erhalten bleiben.

Snap freezing in der Pathologie basiert zumeist auf flüssigem Stickstoff als Kühlmittel und einem Kontaktkryogen wie 2-Methylbutan (Isopentan).

Isopentan ist ein brennbarer Gefahrstoff, der beim Verschlucken schwerste Schädigungen auslösen kann. Daher gilt Isopentan (CAS-NR 78-78-4) als Gefahrstoff und wird nach GHS als entzündlich, gesundheitsgefährdend, reizend und umweltgefährlich gekennzeichnet und muss speziell entsorgt werden. Weiterhin kann der Transport von Isopentan nur in speziellen Transportbehältnissen vorgenommen werden, verbunden mit weiteren Auflagen für die Durchführung derartiger Transporte.

Das Isopentan wird im flüssigen Stickstoff soweit heruntergekühlt, bis es sich bei seinem Schmelzpunkt von -159,9°C verfestigt. Der Abkühlprozess muss hierzu genau beobachtet werden, um zu vermeiden, dass sich das gesamte Isopentan verfestigt, bzw. um zu vermeiden, dass das Isopentan zu warm ist, um die Proben schnell einfrieren zu können.

Die Probe wird bei erreichter Temperatur des Kryogens eingetaucht, bis es durchgefroren ist, und dann in ein geeignetes Gefäß verpackt und im tiefkalten Zustand in einer entsprechend gekühlten Biobank gelagert.

Bei dem oben beschriebenen Verfahren gibt es keine Anhaltspunkte, wann ein Gewebestück durchgefroren ist, da dies sehr stark abhängig von der Gewebegröße ist und bei der oben beschriebenen Methode keine detaillierte Kontrolle des Einfrierprozesses durch den Pathologen erfolgt.

Weiterhin, da es bei den Analysemethoden zur personalisierten oder stratifizierten Medizin wichtig ist, das kranke Gewebe mit gesundem Gewebe des gleichen Patienten zu vergleichen, werden hierfür mehrere Proben eingefroren.

Im oben beschriebenen Verfahren ist eine Standardisierung nicht möglich, so dass es zu Abweichungen durch das Handling kommen kann, die bei der Datenanalyse nicht nachvollzogen werden können.

Des Weiteren fehlen in vielen Pathologien und Krankenhäusern ein Vorrat an flüssigem Stickstoff sowie geeignete Lagermöglichkeiten im tiefkühlen Bereich.

Dementsprechend müssen die in der Operation gewonnenen Proben zu Einrichtungen (im Krankenhaus oder extern) transportiert werden, in denen Snap-Freezing durch standardisierte Snap-Freezing Geräte durchgeführt werden kann. Der Transport zu diesen Einrichtungen führt jedoch zu oben beschriebenen Degradationen, wodurch die Proben nicht mehr für die Anwendung personalisierter oder stratifizierter Medizin verwendet werden können.

Es ist daher die Aufgabe der Erfindung, eine Vorrichtung sowie ein Verfahren zu schaffen, die es ermöglichen, Bioproben schnell tiefzugefrieren.

Die Aufgabe wird gelöst durch eine Vorrichtung für das Kühlen von biologischen und/oder medizinischen Proben, umfassend
- eine Einfriervorrichtung für die biologischen und/oder medizinischen Proben, wobei die Einfriervorrichtung aus vorkühlbaren Teiloberflächenelementen besteht, wobei die vorkühlbaren Teiloberflächenelemente (3a, 3b) aus einem Material mit einer geringen Kälteversprödung bestehen und eine Wärmeleitfähigkeit größer als 200 W/(m•K) aufweisen, wobei wenigstens eines der Teiloberflächenelemente gegenüber dem wenigstens einen weiteren Teiloberflächenelement beweglich angeordnet ist, wobei die Teiloberflächenelemente im zusammengefügten Zustand die Einfriervorrichtung bilden und mit der zwischen den Teiloberflächenelementen befindlichen Probe in thermisch wechselwirkendem Kontakt sind und wobei die Proben im beabstandeten Zustand der Teiloberflächenelemente aus der Einfriervorrichtung fallen, sowie
- eine Auslösevorrichtung, wobei die Auslösevorrichtung einen gespannten Zustand aufweist sowie einen ausgelösten Zustand, wobei die Auslösevorrichtung wenigstens ein Aktivierungsmittel aufweist, durch dessen Betätigung die Auslösevorrichtung von dem gespannten Zustand in den ausgelösten Zustand bringbar ist, wobei die Auslösevorrichtung im gespannten Zustand wenigstens eines der Teiloberflächenelemente von dem wenigstens einen weiteren Teiloberflächenelement beabstandet hält und wobei im ausgelösten Zustand das wenigstens eine bewegliche Teiloberflächenelement und das wenigstens eine weitere Teiloberflächenelement aneinander gedrückt werden.

Die Vorrichtung weist mehrere Betriebszustände auf:
> In einem ersten Betriebszustand der Vorrichtung ist zumindest die Einfriervorrichtung vorgekühlt. Außerdem ist die Auslösevorrichtung im gespannten Zustand.
> In einem zweiten Betriebszustand der Vorrichtung ist zumindest die Einfriervorrichtung vorgekühlt. Die Auslösevorrichtung ist im ausgelösten Zustand.

Das Vorkühlen "zumindest der Einfriervorrichtung" bedeutet, dass auch die Vorrichtung insgesamt vorgekühlt sein kann. Das Vorkühlen zumindest der Einfriervorrichtung gehört zur Vorbereitung der Vorrichtung für den nachfolgenden Gebrauch beim Einfrieren einer Probe.

Der Begriff "Kühlen von biologischen und/oder medizinischen Proben" bedeutet im Rahmen dieser Erfindung, dass die Proben auf eine Temperatur von unter -40°C abgekühlt werden können.

Eine Kühlung der Proben auf einen tiefgefrorenen Bereich (ca. -40°C bis -80°C) ist vorteilhaft für biologische Proben, was die Haltbarkeit bzw. die Verlangsamung des Zerfalls von z.B. Phosphorproteinen bzw. RNA betrifft, wenn man dies mit der Lagerung bei Raumtemperatur oder bei 4°C (Kühlschrank) vergleicht.

Eine Kühlung der Proben auf einen ultratiefkühlen Bereich von unter -130°C ist vorteilhaft für biologische Proben im Vergleich zur Lagerung bei höheren Temperaturen, da dadurch die Funktionalität von Zellen bewahrt wird und der Zerfall bzw. der Abbau essenzieller Moleküle wie z.B. DNA, RNA, Proteine und Effektormoleküle auf ein höchstmögliches Minimum reduziert wird. Der Istzustand der jeweiligen Zelle wird durch die Tiefsttemperatur (unter -130°C) konserviert, so dass die Proben auch nach Jahren ohne qualitative Einbußen auf z.B. Tumormarkern oder anderweitiger diagnostischer oder analytischer Marker hin untersucht werden können.

Die Teiloberflächenelemente bestehen aus vorkühlbarem Material mit einer geringen Kälteversprödung und mit einer hohen Wärmeleitfähigkeit (>200W/(mK)). Es ist daher vorgesehen, dass das Material Aluminium, Kupfer, Gold, Silber oder eine Kombination der vorgenannten Materialien ist. Bei einer Materialauswahl von Materialien, die eine geringere Wärmeleitfähigkeit aufweisen können, kommen auch Edelstahl, insbesondere Titan, in Betracht.

Da wenigstens eines der Teiloberflächenelemente gegenüber dem wenigstens einen weiteren Teiloberflächenelement beweglich angeordnet ist, können die Teiloberflächenelemente zueinander beabstandet voneinander angeordnet werden. Es ist hierbei vorgesehen, dass die Auslösevorrichtung die Teiloberflächenelemente entgegen einer Spann-, Druck- oder Rückhaltekraft (beispielsweise entgegen der Spannkraft einer Feder) voneinander beabstandet halten kann.

Sind die Teiloberflächenelemente vorgekühlt (beispielsweise auf unter -80°C) oder ultratief vorgekühlt (beispielsweise auf -130°C bis zu -190°C) und zueinander beabstandet, kann eine biologische und/oder medizinische Proben zwischen den Teiloberflächenelementen eingebracht werden. Durch Betätigung der Aktivierungsmittel wird die Auslösevorrichtung ausgelöst, d.h. die Auslösevorrichtung wird vom gespannten Zustand in den ausgelösten Zustand überführt.

Durch die bewegliche Anordnung der Teiloberflächenelemente zueinander gehen diese dadurch in den zusammengefügten Zustand über. Die zwischen den Teiloberflächenelementen angeordnete Probe ist dann mit den Teiloberflächenelementen in thermisch wechselwirkendem Kontakt.

Der Bewegungsablauf der Teiloberflächenelemente von dem gespannten Zustand in den ausgelösten Zustand und umgekehrt kann so realisiert sein, dass das wenigstens eine Teiloberflächenelement, das sich dabei bewegt, durch eine Linearführung geführt wird. Vorteilhaft kann auch eine im Wesentlichen punktförmige Lagerung vorgesehen sein, wobei das wenigstens eine Teiloberflächenelement mit einem Hebel an dieser punktförmigen Lagerung befestigt ist. Dabei führt das wenigstens eine Teiloberflächenelement eine Kreisbewegung aus. Vorteilhaft ist das wenigstens eine Teiloberflächenelement so gelagert, dass die Kreisbewegung in horizontaler Richtung verläuft. Damit lässt sich die Vorrichtung so konstruieren, dass nach dem Überführen der Vorrichtung in den gespannten Zustand eine zuvor zwischen den Teiloberflächenelementen eingeklemmte Probe nach unten herausfällt, ohne dass dies durch einen Hebel des Teiloberflächenelementes gestört wird.

Die Geschwindigkeit, mit der die Teiloberflächenelemente in den ausgelösten Zustand übergehen, lässt sich erhöhen, wenn beide Teiloberflächenelemente bewegt werden.

Der thermisch wechselwirkende Kontakt zwischen der Probe und den Teiloberflächenelementen wird vorzugsweise durch einen direkten körperlichen Kontakt von wenigstens einer Fläche eines der Teiloberflächenelemente und der Probe erreicht.

Es ist weiterhin vorteilhaft vorgesehen, dass die Probe zwischen den Teiloberflächenelementen eingeklemmt ist oder zwischen die Teiloberflächenelemente gedrückt oder gepresst wird. Das Klemmen, Pressen oder Drücken wird durch die ausgelöste Auslösevorrichtung erreicht. Durch den physischen Kontakt der Teiloberflächenelemente mit der Probe wird vorteilhaft erreicht, dass thermische Energie ausgetauscht wird, so dass die Temperatur der Probe schnell (< 1 s) die Temperatur der Teiloberflächenelemente annimmt.

Durch die Vorrichtung für das Kühlen von biologischen und/oder medizinischen Proben wird damit vorteilhaft erreicht, dass die Probe an jedem Ort, d.h. auch in einem Operationssaal (OP), in der Ambulanz, in einem Labor oder bei einem Feldversuch tiefgefroren (Kühlung der Probe auf einen tiefgefrorenen Bereich; ca. -40°C bis -80°C) oder ultratiefgefroren (Kühlung der Probe auf einen ultratiefkühlen Bereich; unter -130°C) werden kann.

Es ist vorteilhaft für die Vorrichtung für das Kühlen von biologischen und/oder medizinischen Proben vorgesehen, dass die vorkühlbaren Teiloberflächenelemente aus Aluminium, Silber, Kupfer oder Gold bestehen.

Aluminium erweist sich als besonders vorteilhaft, weil es mit 237 W/(m·K) eine hohe Wärmeleitfähigkeit aufweist und außerdem eine geringe Tieftemperaturversprödung aufweist. Außerdem weist Aluminium ein geringes spezifisches Gewicht auf. Dies ist vorteilhaft für das Gesamtgewicht der Vorrichtung. Dies zeigt sich insbesondere dann, wenn die Vorrichtung transportiert werden soll.

Damit man die eventuell tiefgefrorene oder ultratiefkühle Vorrichtung für das Kühlen von biologischen und/oder medizinischen Proben nicht manuell auslösen muss, ist zu der Erfindung gehörig, dass das wenigstens eine Aktivierungsmittel der Auslösevorrichtung zur Überführung der Auslösevorrichtung von dem gespannten Zustand in den gelösten Zustand durch einen mechanischen und/oder magnetischen und/oder elektromagnetischen und/oder elektrischen und/oder optischen Auslöser ausgestaltet wird.

Beispielsweise kann der Chirurg oder Laborant die Probe über der gespannten Vorrichtung (vertikal zu dem freien Bereich zwischen den gespannten Teiloberflächenelementen) loslassen, wobei die Probe aufgrund der Erdanziehungskraft nach unten, zwischen die Teiloberflächenelemente, fällt und beispielsweise eine Lichtschranke passiert. Dieses Passieren der Lichtschranke löst das wenigstens eine Aktivierungsmittel der Auslösevorrichtung aus. Ebenso ist vorstellbar, dass der Arzt mit einer Hand die Probe fallen lässt und mit der anderen Hand einen externen mechanischen oder magnetischen Auslöser bedient.

Durch einen mechanischen und/oder magnetischen und/oder elektromagnetischen und/oder elektrischen und/oder optischen Auslöser wird verhindert, dass der Arzt mit seiner Hand in den sehr kalten oder ultratiefkühlen Bereich der Auslösevorrichtung greifen muss, während die Auslösevorrichtung ausgelöst wird. Durch die Ausgestaltung der Vorrichtung mit einem Auslöser wird daher die Arbeitssicherheit erhöht.

Ebenso ist vorteilhaft vorgesehen, dass wenigstens ein Spannmittel vorhanden ist zur Überführung der Auslösevorrichtung von dem ausgelösten Zustand in den gespannten Zustand durch einen mechanischen und/oder magnetischen und/oder elektromagnetischen und/oder elektrischen Spannmechanismus.

Das Betätigen des Spannmechanismus wird vorteilhaft dadurch erreicht, dass an der Vorrichtung Mittel angeordnet sind, durch die der Spannmechanismus angetrieben wird. Durch diese Ausgestaltung wird ebenfalls das Verletzungsrisiko vermindert, da die Teiloberflächenelemente über den Spannmechanismus - und eben nicht manuell - voneinander beabstandet werden.

Damit sichergestellt ist, dass die Probe zwischen die Teiloberflächenelemente gelangt, ist weiterhin vorteilhaft vorgesehen, dass der Vorrichtung zum Kühlen wenigstens eine Führungsvorrichtung zum gleitenden Einführen der Probe in die Vorrichtung zugeordnet ist.

Die Führungsvorrichtung zum gleitenden Einführen der Probe ist vorzugsweise eine längliche Vorrichtung, die im Querschnitt als eckiger oder runder Zylinder, eckiger oder runder Hohlzylinder oder U-, V- oder L-förmig ausgebildet sein kann. Durch die Ausgestaltung im Querschnitt als eckiger oder runder Zylinder, eckiger oder runder Hohlzylinder oder U-, V- oder L-förmig wird erreicht, dass die Probe gegen ein seitliches Verrutschen gesichert ist. Es kann weiterhin vorgesehen sein, dass die Führungsvorrichtung zum gleitenden Einführen schräg oder im Wesentlichen vertikal zu dem Bereich zwischen den Teiloberflächenelementen im gespannten Zustand der Auslösevorrichtung angeordnet werden kann. Soll die Probe im Wesentlichen vertikal zwischen die Teiloberflächenelementen im gespannten Zustand eingebracht werden, ist vorteilhaft vorgesehen, dass die Führungsvorrichtung im Querschnitt als eckiger oder runder Hohlzylinder ausgebildet ist oder dass zwei Führungsvorrichtungen verwendet werden, die im Querschnitt U-, V- oder L-förmig ausgebildet sind, wobei die offenen Bereiche der Querschnitte einander zugewandt sind.

Die Führungsvorrichtung kann an der Vorrichtung demontierbar angebracht sein, beispielsweise durch eine Steckverbindung. Dies erweist sich insofern als vorteilhaft, weil damit einer in einem Kryobehälter befindlichen Vorrichtung eine Probe zugeführt werden kann, die außerhalb des Kryobehälters in die Führungsvorrichtung eingebracht wird. Dies gilt insbesondere, wenn die Führungsvorrichtung so lang ist, dass diese im montierten Zustand aus dem Kryobehälter herausragt. Es ist insbesondere nicht notwendig, in den Kryobehälter hineinzugreifen. Durch die Demontierbarkeit wird es vorteilhaft möglich, nach dem Einbringen der Probe die Führungsvorrichtung zu entfernen, so dass der Kryobehälter dann verschlossen werden kann. Dies erweist sich insbesondere bei einem Behälter nach Anspruch 10 als vorteilhaft.

Für Aufbewahrungs-, Sterilitäts- und Lagerungszwecke ist weiterhin vorteilhaft zu der Erfindung gehörig, dass die biologische und/oder medizinische Probe in einem Probenbehälter, insbesondere einer Tüte, einer Kanüle oder einem Röhrchen (z.B. verschließbares Kryoröhrchen), anordbar ist.

Der Probenbehälter ist vorzugsweise ein ultratiefkühltauglicher Probebehälter, der als Tüte, Kanüle oder Röhrchen ausgebildet sein kann. Im Falle, dass der Probenbehälter als Tüte ausgebildet ist, kann vorgesehen sein, dass ein Gewicht an dem der Öffnung der Tüte gegenüberliegenden Bereich angeordnet ist. Das Gewicht ist vorteilhaft, um zu erreichen, dass sich die Tüte einfacher mit der Probe befüllen lässt und die Probe mit dem Gewicht voran zwischen die gespannten Teiloberflächenelemente eingebracht werden kann.

Es ist weiterhin vorteilhaft vorgesehen, dass der Probenbehälter, insbesondere eine Tüte, eine Kanüle oder ein Röhrchen, in oder an einer Probenkassette anordbar ist, wobei die Probenkassette derart ausgestaltet ist, dass sie durch die wenigstens eine zuvor beschriebene Führungsvorrichtung führbar ist.

Beispielsweise kann die Probenkassette aus einem Metallrahmen bestehen (beispielsweise aus Aluminium), durch den ein Probenbehälter, der insbesondere eine Tüte, Kanüle oder ein Röhrchen sein kann, mit einer darin enthaltenen Probe eingespannt wird.

Die Tüte erweist sich insofern als vorteilhaft, weil dadurch keine großen Luftvolumina entstehen und die Tüte faltenfrei einspannbar ist. Es ergibt sich eine einfache Bedienung für den Verschluss der Tüte (beispielsweise durch Clips). Die Probenkassette kann eine Aufnahmemöglichkeit für verschiedene gängige Tütenformate von z.B. 40x40 mm bis 70 bis 120 mm aufweisen, um eine optimierte Positionierung der Probe für den Kühlvorgang gewährleisten zu können.

Eine Ausgestaltung der Erfindung sieht weiter vorteilhaft vor, dass das wenigstens eine Aktivierungsmittel der Auslösevorrichtung zur Überführung der Auslösevorrichtung von dem gespannten Zustand in den gelösten Zustand durch die Probenkassette aktivierbar ist.

Beispielsweise kann an der Probenkassette ein abstehendes Mittel angeordnet sein, dass beim Herabfallen auf einen mechanischen, magnetischen, elektromagnetischen oder elektrischen Auslöser trifft, wodurch die Auslösevorrichtung ausgelöst wird.

Eine weitere Ausgestaltung der Erfindung sieht vor, dass das wenigstens eine Spannmittel der Auslösevorrichtung zur Überführung der Auslösevorrichtung von dem ausgelösten Zustand in den gespannten Zustand durch einen mechanischen Spannmechanismus realisiert ist, wobei das Mittel zum mechanischen Spannen des Spannmechanismus zugleich die Führungsvorrichtung zum gleitenden Einführen der Probe in die Vorrichtung ist.

In diesem Zusammenhang wird insbesondere auf die Vorteile einer demontierbaren Führungsvorrichtung verwiesen, die im Zusammenhang mit Anspruch 5 erläutert sind. Bei der Ausgestaltung nach Anspruch 9 bedeutet dies, dass dann auch die Spannmittel außerhalb des Kryobehälters betätigt werden können und nach dem Einbringen der Probe entfernt werden können.

Das Spannen des Spannmechanismus erfolgt beispielsweise dadurch, dass die Führungsvorrichtung mit dem Spannmechanismus verbunden wird. Durch eine Rotationsbewegung der Führungsvorrichtung wird der Spannmechanismus gespannt.

Nachfolgend wird das Verfahren zum Betreiben der zuvor beschriebenen Vorrichtung für das Kühlen von biologischen und/oder medizinischen Proben beschrieben.

Das Verfahren zum Betreiben der Vorrichtung für das Kühlen von biologischen und/oder medizinischen Proben, umfasst die Schritte:
a) Kühlen der Einfriervorrichtung oder der gesamten Vorrichtung für das Kühlen
b) Spannen der Auslösevorrichtung,
c) Einbringen der Probe zwischen die Teiloberflächenelemente der Einfriervorrichtung,
d) Überführen der Auslösevorrichtung vom gespannten Zustand in den ausgelösten Zustand.

Ebenso kann das Verfahren zum Betreiben der Vorrichtung für das Kühlen von biologischen und/oder medizinischen Proben durch die Schritte
a) Kühlen der Einfriervorrichtung oder der gesamten Vorrichtung für das Kühlen
b) Spannen der Auslösevorrichtung,
c) Einbringen der Probe zwischen die Teiloberflächenelemente der Einfriervorrichtung und dadurch bedingtes Überführen der Auslösevorrichtung vom gespannten Zustand in den ausgelösten Zustand
   erfolgen.

Für beide zuvor beschriebenen Verfahren ist erfindungsgemäß vorgesehen, dass nach Schritt c) oder Schritt d) Schritt b) wiederholt werden kann. Weiterhin kann vorgesehen sein, dass nach dem Schritt b) eine Positionierung einer Führungsvorrichtung durchgeführt wird. Ggf. kann diese Führungsvorrichtung auch bereits nach Schritt a) positioniert werden, wenn diese Führungsvorrichtung zugleich auch zum Spannen der Auslösevorrichtung verwendet wird.

Ebenso wird die Aufgabe durch eine Vorrichtung für das Einfrieren und Kühlhalten von biologischen und/oder medizinischen Proben gelöst, wobei die Vorrichtung
- einen Behälter mit einem Außengehäuse mit einer Öffnung und mit einer porösen Innenauskleidung sowie einem Deckel zum Verschließen der Öffnung und
- eine in das Innenvolumen des Behälters einbringbare oder in dem in das Innenvolumen des Behälters festangeordnete zuvor beschriebene Vorrichtung für das Kühlen von biologischen und/oder medizinischen Proben
   umfasst.

Es ist hierfür zu der Erfindung gehörig, dass das Außengehäuse des Behälters eine höchstmögliche Isolation erbringt. Dieses wird z.B. durch Vakuumisolationstechnik erreicht.

Vorteilhaft ist das Material des Außengehäuses bruchsicher und weist eine ausreichende Festigkeit gegenüber Stößen auf, die beim Transport üblicherweise auftreten können.

Durch die Maßnahmen können die Dimension und das Gewicht der Vorrichtung derart gestaltet sein, dass sie durch eine Person tragbar ist. Hierfür ist vorteilhaft vorgesehen, dass an dem Außengehäuse Mittel zum Anheben der Vorrichtung, wie Griffe oder Einbuchtungen des Außengehäuses, angeordnet sind. Durch diese Ausgestaltung kann die Vorrichtung einfach angehoben, getragen und transportiert werden.

Weiterhin ist vorteilhaft vorgesehen, dass der Behälter eine Öffnung aufweist, durch die Proben oder andere Gegenstände (z.B. die Vorrichtung für das Kühlen von biologischen und/oder medizinischen Proben) eingebracht werden können.

Es ist weiterhin vorgesehen, dass der Behälter eine poröse bzw. absorbierende Innenauskleidung aufweist, die ein Kältemittel, vorzugsweise Flüssigstickstoff, durch Adhäsion bindet. Die poröse bzw. absorbierende Innenauskleidung besteht vorzugsweise aus Silikaten, welche das flüssige Gas binden und gleichzeitig leicht sind. Die Innenauskleidung ist derart in dem Behälter angeordnet, dass ein durch die Öffnung zugängliches Innenvolumen frei bleibt. Dadurch, dass das Kältemittel gebunden ist, kann die Vorrichtung ohne Gefahr des plötzlichen Auslaufens transportiert werden. Weiterhin kann die Vorrichtung, da sie ein Kältemittel binden kann, eine Probe kühlhalten. Dementsprechend benötigt die Vorrichtung keine externe Energieversorgung, um die Kühlung zu erreichen, sondern ist - für einen definierten Zeitraum von beispielsweise mehreren Tagen - Netz-/Strom unabhängig.

Zwischen dem Außengehäuse und der Innenauskleidung ist weiterhin vorteilhaft die bereits beschriebene und dem Fachmann bekannte Vakuumisolierung angeordnet.

Weiterhin ist vorgesehen, dass ein Deckel die Öffnung des Behälters kraft- und/oder formschlüssig verschließen kann. Der Formschluss kann beispielsweise durch eine auf der Innenseite des Deckels hervorstehende Styroporschicht erreicht werden, wobei die Außenmaße der Styroporschicht im Wesentlichen den Innenmaßen der Öffnung entsprechen. Der Kraftschluss kann beispielsweise durch ein Außengewinde an der Öffnung und ein mit dem Außengewinde der Öffnung korrespondierendem Gewinde an dem Deckel erreicht werden.

Weiterhin ist vorgesehen, dass in dem Innenvolumen des Behälters eine Vorrichtung für das Kühlen von biologischen und/oder medizinischen Proben fest angeordnet sein kann. Ebenso ist zu der Erfindung gehörig, dass eine Vorrichtung für das Kühlen von biologischen und/oder medizinischen Proben durch die Öffnung des Behälters in diesen einbringbar ist.

Durch die Vorrichtung für das Einfrieren und Kühlhalten von biologischen und/oder medizinischen Proben wird erreicht, dass Proben in der klinischen Routineversorgung von Patienten für molekulardiagnostische Anwendungen bereits im Operationssaal (OP), in der Ambulanz, bei Probenentnahmen (z.B. Darmspiegelung mit Gewebeproben bzw. Flüssigbiopsien wie z.B. Blut, Sputum oder Liquor) und/oder beim Pathologen ohne die Verwendung von Flüssigstickstoff im Operationssaal oder in der Ambulanz schnell tiefgefroren werden können. Weiterhin wird durch diese Vorrichtung erreicht, dass die Proben in dem tiefgefrorenen Zustand transportiert werden können.

Es ist beispielsweise vorgesehen, dass die Vorrichtung außerhalb des Operationssaals oder der Ambulanz vorbereitet wird, dann zu dem Probenentnahmeeinsatzort transportiert wird, wo die Probe entnommen und in der Vorrichtung tiefgefroren wird und die Vorrichtung nach Beendigung der Probenentnahme daraufhin zu einer Lagerstätte oder zu einem Labor für die Durchführung histologischer Untersuchungen transportiert wird.

Für die Vorbereitung der Vorrichtung für das Einfrieren und Kühlhalten von biologischen und/oder medizinischen Proben ist ein Verfahren mit den folgenden Schritten vorgesehen:
a) Einbringen der Vorrichtung für das Kühlen in das Innenvolumen des Behälters und
b) Fluten oder Laden oder Sättigen des Innenvolumens (und damit die poröse Innenauskleidung) und der Vorrichtung für das Kühlen mit Flüssigstickstoff oder einem anderen Kühlmittel.

Alternativ ist für die Vorbereitung der Vorrichtung für das Einfrieren und Kühlhalten von biologischen und/oder medizinischen Proben ein Verfahren mit den folgenden Schritten vorgesehen:
a) Fluten oder Laden oder Sättigen des Innenvolumens (und damit die poröse Innenauskleidung) mit Flüssigstickstoff oder einem anderen Kühlmittel und
b) Einbringen der Vorrichtung für das Kühlen in das Innenvolumen des Behälters.

Es ist vorgesehen, dass die Temperatur im Innenraum beispielsweise zwischen 3 Tagen und 14 Tagen gehalten wird.

Die vorbereitete Vorrichtung für das Einfrieren und Kühlhalten von biologischen und/oder medizinischen Proben kann daraufhin zu dem Probenentnahmeort transportiert werden. Eine netzabhängige, d.h. stromabhängige Versorgung ist nicht vonnöten. Es ist möglich, mit der Vorrichtung eine Probe tiefzukühlen und unmittelbar anschließend in dem Behälter zu lagern für einen Zeitraum von beispielsweise mehreren Tagen.

Ist die Vorrichtung für das Einfrieren und Kühlhalten am Probenentnahmeort angekommen, können die Proben durch den Arzt oder Chirurg von dem Patienten entnommen werden und mittels der Vorrichtung für das Kühlen, die in der Vorrichtung für das Einfrieren und Kühlhalten angeordnet ist, tiefgefroren oder ultratiefgekühlt werden.

Dementsprechend wird die Aufgabe ebenfalls durch ein Verfahren zum Einfrieren und Kühlhalten von biologischen und/oder medizinischen Proben unter Verwendung einer zur Durchführung des folgenden Verfahrens vorbereiteten Vorrichtung zum Einfrieren und Kühlhalten gelöst, wobei das Verfahren die Schritte
a) Einbringen einer biologischen und/oder medizinischen Probe in einen Probenbehälter,
b) Öffnen des Deckels zum Verschließen der Öffnung des Behälters,
c) Spannen der Auslösevorrichtung der Vorrichtung zum Kühlen,
d) Einbringen des mit der biologischen und/oder medizinischen Probe versehenen Probenbehälters zwischen die Teiloberflächenelemente der Einfriervorrichtung,
e) Überführen der Auslösevorrichtung vom gespannten Zustand in den ausgelösten Zustand,
f) Warten
g) Überführen der Auslösevorrichtung vom ausgelösten Zustand in den gespannten Zustand und/oder
h) Einsetzen des Deckels zum Verschließen der Öffnung des Behälters
   umfasst.

Es ist ebenfalls möglich, einzelne der Verfahrensschritte zusammenzufassen. Dies gilt beispielsweise für die Schritte d) und e), so dass sich dann folgende Schritte für das Verfahren ergeben:
a) Einbringen einer biologischen und/oder medizinischen Probe in einen Probenbehälter,
b) Öffnen des Deckels zum Verschließen der Öffnung des Behälters,
c) Spannen der Auslösevorrichtung der Vorrichtung zum Kühlen,
d) Einbringen des mit der biologischen und/oder medizinischen Probe versehenen Probenbehälters zwischen die Teiloberflächenelemente der Einfriervorrichtung und dadurch bedingtes Überführen der Auslösevorrichtung vom gespannten Zustand in den ausgelösten Zustand,
e) Warten
f) Überführen der Auslösevorrichtung vom ausgelösten Zustand in den gespannten Zustand und/oder
g) Einsetzen des Deckels zum Verschließen der Öffnung des Behälters.

Für beide vorgenannte Verfahren wird die Probe durch die die Probennahme durchführende Person entnommen, wonach die Probe in ein Probenbehältnis, beispielsweise eine Tüte, eingebracht wird. Ebenso ist zu der Erfindung gehörig, dass die Probe mit einer Kanüle entnommen wird, wodurch die Probe dann bei der Entnahme in ein Probenbehältnis, nämlich in die Kanüle, eingebracht wird.

Nach dem Öffnen des Deckels zum Verschließen der Öffnung des Behälters, wird die Vorrichtung zum Kühlen gespannt, so dass die beiden Teiloberflächenelemente der Einfriervorrichtung der Vorrichtung zum Kühlen, die in der netzunabhängigen / autarken tragbaren Vorrichtung angeordnet ist, voneinander beabstandet sind.

Das Spannen der Auslösevorrichtung der Vorrichtung zum Kühlen kann wie zuvor beschrieben durchgeführt werden. Ebenso werden die Schritte des Positionierens des mit der biologischen und/oder medizinischen Probe versehenen Probenbehälters zwischen die Teiloberflächenelemente der Einfriervorrichtung der Vorrichtung zum Kühlen sowie das Auslösen bzw. Überführen der Auslösevorrichtung vom gespannten Zustand in den ausgelösten Zustand entsprechend der zuvor beschriebenen Möglichkeiten durchgeführt.

Der Schritt "Warten", bei dem die Probe eingefroren wird, kann wenige Sekunden, vorzugsweise sogar weniger als eine Sekunde, dauern. Durch den Wärmeaustausch der tiefgefrorenen oder ultratiefgefrorenen Teiloberflächenelemente und der Probe wird die Probe sehr schnell komplett durchgefroren. Dies ist vorteilhaft, da biologische Prozesse innerhalb der Zellen sehr schnell auf die Kälteeinwirkung reagieren und somit Ischämie verhindert wird.

Durch die Vorrichtung für das Einfrieren und Kühlhalten und das Verfahren zum Einfrieren und Kühlhalten von biologischen und/oder medizinischen Proben unter Verwendung der Vorrichtung kann der Umgang mit Gefahrstoffen wie Isopentan oder flüssigem Stickstoff an dem Probenentnahmeort (OP oder Ambulanz) vermieden werden, wodurch ein verbesserter Arbeitsschutz gegeben ist.

Gerade kleineren Pathologien bzw. sogar den Chirurgen selbst wird damit ermöglicht, Tumorgewebe oder Flüssigbiopsien tiefzugefrieren oder ultratief einzufrieren. Diese Proben können nachfolgend in einer Biobank gelagert und somit für weitere diagnostische Verfahren zugänglich gemacht werden.

Weiterhin kann durch die Verwendung der vorgenannten Vorrichtungen und Verfahren eine Standardisierung des Einfrierens erreicht werden, so dass die Abweichungen zwischen den einzelnen Proben minimiert werden, wodurch weiterhin eine bessere Vergleichbarkeit realisiert wird.

Nachfolgend wird die Erfindung anhand von Zeichnungen näher erläutert, aber nicht auf die gezeigte Ausführungsform beschränkt. Es zeigen
- Fig. 1: eine schematische Perspektivansicht einer Vorrichtung für das Kühlen von biologischen und/oder medizinischen Proben, umfassend eine Einfriervorrichtung für die biologischen und/oder medizinischen Proben und eine Auslösevorrichtung,
- Fig. 2: eine schematische Perspektivansicht einer Vorrichtung für das Kühlen von biologischen und/oder medizinischen Proben mit einer Einfriervorrichtung für die biologischen und/oder medizinischen Proben und eine durch die Führungsvorrichtung gespannte Auslösevorrichtung,
- Fig. 3: eine schematische Draufsicht der Vorrichtung aus Fig. 2,
- Fig. 4: eine schematische Perspektivansicht der Vorrichtung aus Fig. 2, wobei eine Probe in einem Probenbehälter angeordnet ist und der Probenbehälter an bzw. in einer Probenkassette angeordnet ist, wobei die Probenkassette und damit auch die Probe durch die beiden Führungsvorrichtungen zu dem Bereich zwischen den Teiloberflächenelementen geführt wird,
- Fig. 5: eine schematische Perspektivansicht einer Vorrichtung für das Kühlen von biologischen und/oder medizinischen Proben mit einer ausgelösten Auslösevorrichtung und einer zwischen den Teiloberflächenelementen angeordneten Probe,
- Fig. 6: eine schematische Draufsicht der Vorrichtung aus Fig. 5,
- Fig. 7: einen Schnitt durch eine schematische Perspektivansicht einer Vorrichtung für das Einfrieren und Kühlhalten von biologischen und/oder medizinischen Proben, umfassend einen Behälter und eine in das Innenvolumen des Behälters eingebrachte Vorrichtung für das Kühlen von biologischen und/oder medizinischen Proben,
- Fig. 8: einen Schnitt durch eine schematische Perspektivansicht der netzunabhängigen tragbaren Vorrichtung aus Fig. 7, wobei der Deckel zum Verschließen der Öffnung nicht den Behälter verschließt,
- Fig. 9: eine schematische Perspektivansicht der Vorrichtung für das Einfrieren und Kühlhalten, wobei in der netzunabhängigen tragbaren Vorrichtung eine Vorrichtung für das Kühlen von biologischen und/oder medizinischen Proben angeordnet ist und eine Probe, die in einer Tüte in einer Probenkassette angeordnet ist, durch die beiden Führungsvorrichtungen zu der Vorrichtung für das Kühlen einbringbar ist.

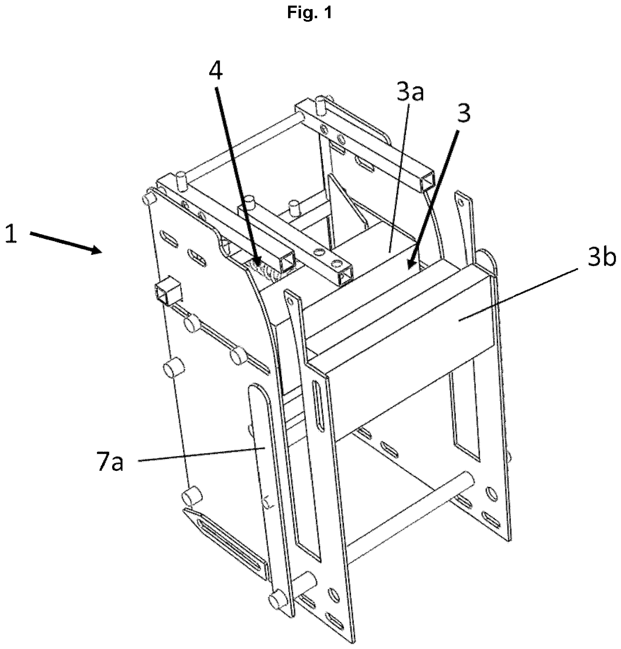

In Fig. 1 ist eine Vorrichtung 1 für das Kühlen von biologischen und/oder medizinischen Proben in schematischer Perspektivansicht gezeigt.

Die Vorrichtung 1 umfasst eine Einfriervorrichtung 3 für biologische und/oder medizinische Proben und eine Auslösevorrichtung 4. Die Einfriervorrichtung 3 für die biologischen und/oder medizinischen Proben besteht aus vorkühlbaren Teiloberflächenelementen 3a, 3b.

Die vorkühlbaren Teiloberflächenelemente 3a, **3b** bestehen aus Aluminium. Es ist ebenfalls möglich, dass die vorkühlbaren Teiloberflächenelemente 3a, **3b** aus Silber, Kupfer oder Gold bestehen.

Wenigstens eines der Teiloberflächenelemente 3a ist gegenüber dem wenigstens einen weiteren Teiloberflächenelement **3b** beweglich angeordnet. Durch diese Ausgestaltung wird ermöglicht, dass die Teiloberflächenelemente 3a, **3b** im zusammengefügten Zustand die Einfriervorrichtung 3 bilden und mit einer zwischen den Teiloberflächenelementen befindlichen Probe in thermisch wechselwirkendem Kontakt sind.

Wie dargestellt, können die Proben im beabstandeten Zustand der Teiloberflächenelemente 3a, **3b** sowohl in den Bereich zwischen den Teiloberflächenelemente 3a, **3b** eingebracht werden als auch aus der Einfriervorrichtung 3 fallen, sollten sie zuvor zwischen den Teiloberflächenelementen 3a, **3b** gepresst worden sein. Für die Anordnung der Teiloberflächenelemente 3a, **3b** wird die Auslösevorrichtung 4 genutzt.

Wie dargestellt, wird die Auslösevorrichtung **4** durch einen mechanischen Spannmechanismus, hier eine Feder, gespannt. Die Auslösevorrichtung **4** hält im gespannten Zustand eines der Teiloberflächenelemente **3a** von dem wenigstens einen weiteren Teiloberflächenelement **3b** beabstandet (s. Fig. 1). Im ausgelösten Zustand werden das wenigstens eine bewegliche Teiloberflächenelement **3a** und das wenigstens eine weitere Teiloberflächenelement **3b** aneinandergedrückt.

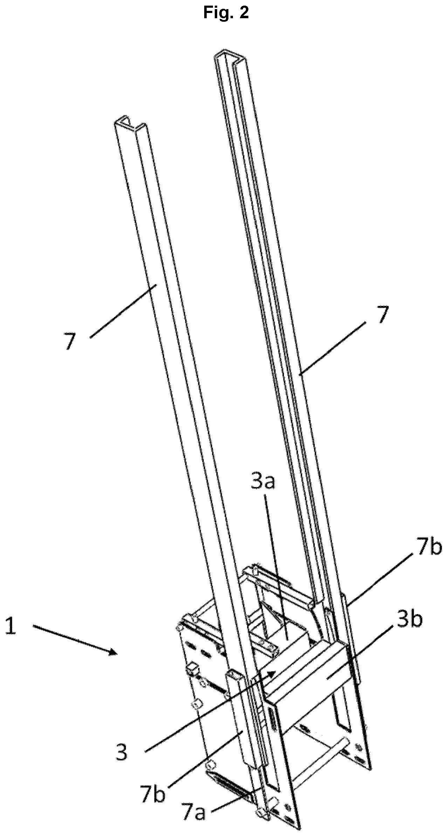

Es sind weiterhin zwei Aufsteckzapfen **7a** zu sehen, zur Aufnahme einer Befestigungshülse der Führungsvorrichtung **7,** die in Fig. 2 näher gezeigt ist.

In Fig. 2 ist eine Vorrichtung **1** für das Kühlen von biologischen und/oder medizinischen Proben mit zwei Führungsvorrichtungen **7** zum gleitenden Einführen einer Probe zwischen die gespannten Teiloberflächenelemente **3a, 3b** in schematischer Perspektivansicht gezeigt.

Durch die Führungsvorrichtungen **7** kann die Auslösevorrichtung mechanisch durch einen Spannmechanismus gespannt werden.

Zugleich ist vorgesehen, dass die Führungsvorrichtungen **7** zum gleitenden Einführen einer Probe zwischen die gespannten Teiloberflächenelemente **3a, 3b** verwendet werden.

Damit die Probe sicher geführt werden kann, ist vorgesehen, dass die Probe in einem Probenbehälter angeordnet ist und der Probenbehälter an oder in einer Probenkassette angeordnet ist. Die Dimensionen der Probenkassette sind derart, dass die Probenkassette gleitend zwischen den Führungsvorrichtungen **7** geführt werden kann.

Die beiden Führungsvorrichtungen **7** sind im Querschnitt U-förmig ausgebildet und derart an der Vorrichtung **1** angeordnet, dass die offenen Seiten des Us einander zugewannt sind. Durch diese Anordnung wird ermöglicht, dass die Probenkassette nicht horizontal verrutschen kann.

Es sind wieder einer der beiden Zapfen **7a** zu sehen sowie die an der Führungsvorrichtung **7** angebrachte Hülse **7b** zum Aufstecken auf den Zapfen **7a.** Dadurch ist die Führungsvorrichtung **7** demontierbar an der Vorrichtung 1 befestigt.

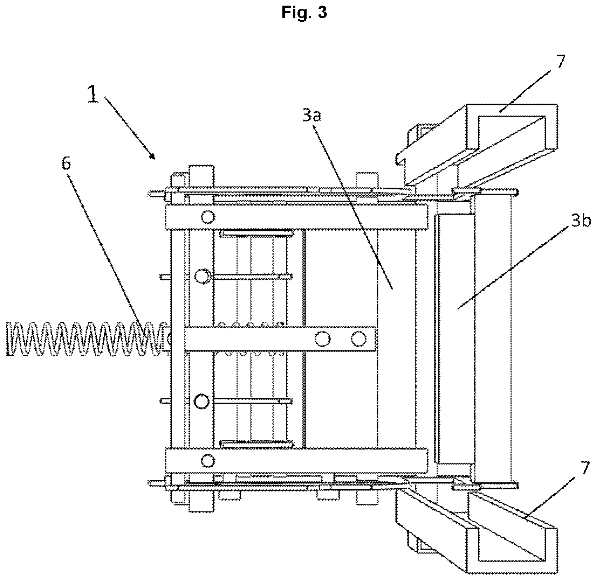

In Fig. 3 ist eine schematische Draufsicht der Vorrichtung **1** aus Fig. 2 gezeigt.

Neben den Führungsvorrichtungen **7** ist der Spannmechanismus **6,** hier ein durch eine Feder umgesetzter mechanischer Spannmechanismus, gezeigt.

Die Führungsvorrichtungen **7** sind an der Vorrichtung **1** angesteckt derart, dass eine Rotationsbewegung wenigstens einer der Führungsvorrichtungen **7** an dem jeweiligen Ansteckpunkt die Feder spannt. Die Feder **6** spannt daraufhin das beweglich angeordnete Teiloberflächenelement **3a** derart, dass das beweglich angeordnete Teiloberflächenelement 3a von dem weiteren Teiloberflächenelement **3b** beabstandet gehalten ist. Weiterhin ist ein Rastelement vorgesehen, das das Teiloberflächenelement **3a** in dieser Position hält. Die Auslösevorrichtung funktioniert derart, dass das Rastelement entfernt wird. Dadurch wird das Teiloberflächenelement **3a** durch die Federkraft an das andere Teiloberflächenelement **3b** gepresst.

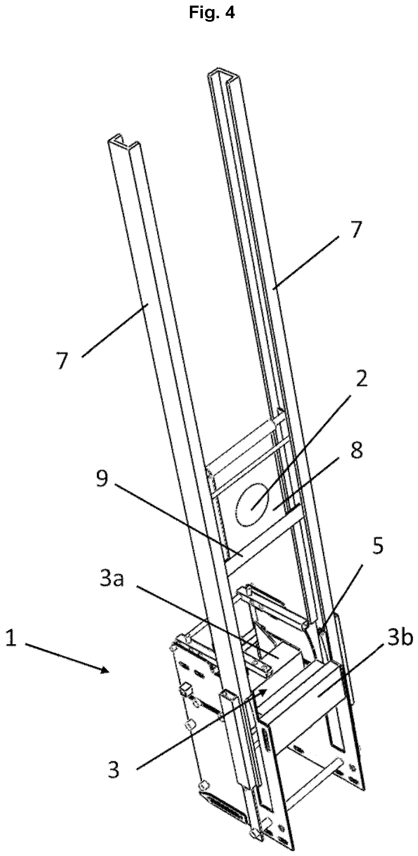

In Fig. 4 ist eine schematische Perspektivansicht der Vorrichtung **1** aus Fig. 2 gezeigt, wobei eine Probe **2** in einem Probenbehälter **8** angeordnet ist und der Probenbehälter **8** an bzw. in einer Probenkassette **9** angeordnet ist.

Die Probenkassette **9** und damit auch die Probe **2** wird durch die beiden Führungsvorrichtungen **7** zu dem Bereich zwischen den Teiloberflächenelementen **3a, 3b** geführt. Weiterhin ist ein Auslöser **5** gezeigt, der die Auslösevorrichtung auslöst, sobald die Probenkassette **9** den Auslöser **5** passiert.

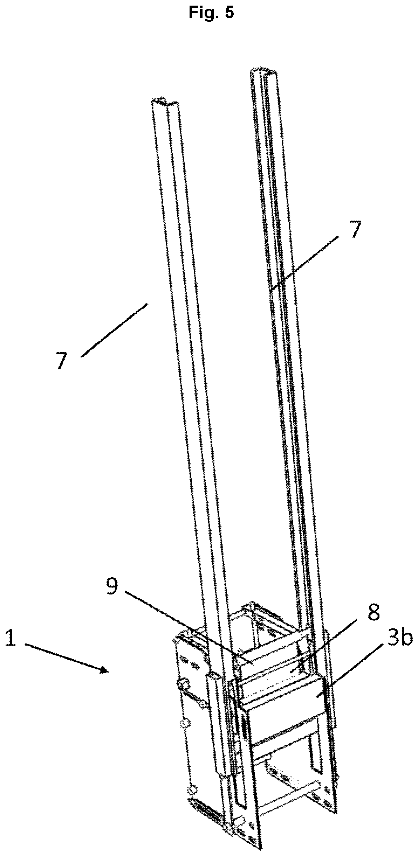

In Fig. 5 ist eine schematische Perspektivansicht der Vorrichtung **1** mit einer ausgelösten Auslösevorrichtung und einer zwischen den Teiloberflächenelementen **3a, 3b** angeordneten Probe gezeigt. Die Probe, die in einem Probenbehälter **8** angeordnet ist, wird zwischen den Teiloberflächenelementen **3a, 3b** gepresst gehalten. Dabei ist der Probenbehälter **8** an bzw. in einer Probenkassette **9** angeordnet.

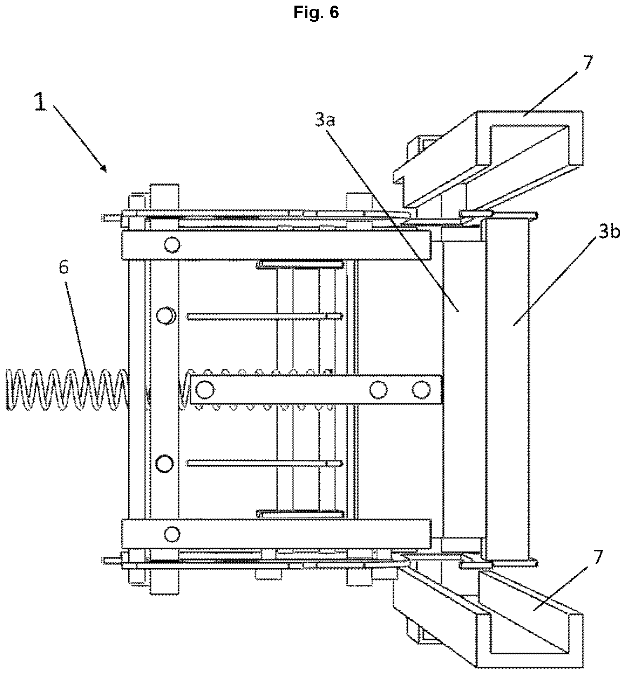

In Fig. 6 ist eine schematische Draufsicht der Vorrichtung **1** aus Fig. 5 gezeigt.

Die Probenkassette mit dem Probenbehälter und der darin angeordneten Probe sind nicht dargestellt.

Neben den Führungsvorrichtungen **7** ist der Spannmechanismus **6,** hier ein durch eine Feder umgesetzter mechanischer Spannmechanismus, gezeigt. Die Feder weist im gespannten Zustand eine größere Federkraft auf als im ausgelösten Zustand.

Auch im ausgelösten Zustand hat die Feder eine verbleibende Federkraft, durch die das Teiloberflächenelement **3a** gegen das Teiloberflächenelement **3b** gepresst wird. Da der Auslösemechanismus ausgelöst ist, sind das eine bewegliche Teiloberflächenelement **3a** und das weitere Teiloberflächenelement **3b** aneinandergedrückt dargestellt.

Da die Teiloberflächenelemente **3a, 3b** vorgekühlt bzw. ultratiefgefroren sind, findet ein thermisch wechselwirkender Austausch zwischen den Teiloberflächenelementen **3a, 3b** und der zwischen den Teiloberflächenelementen **3a, 3b** angeordneten Probe statt. Die Probe wird daher durch den thermisch wechselwirkenden Kontakt tiefgefroren.

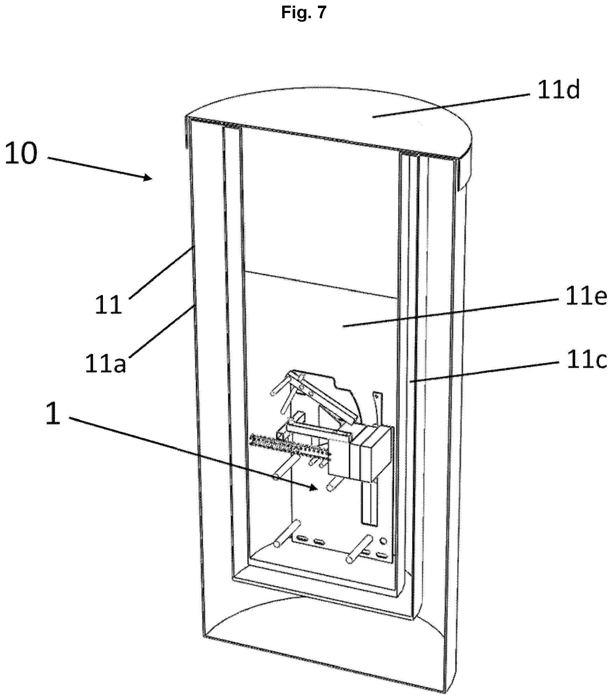

In Fig. 7 ist ein Schnitt durch eine schematische Perspektivansicht einer netzunabhängigen (d.h. autarken) tragbaren Vorrichtung **10** für das Einfrieren bzw. schnelle Ultratiefkühlen und Kühlhalten von biologischen und/oder medizinischen Proben gezeigt.

Die netzunabhängige tragbare Vorrichtung **10** umfasst einen Behälter **11,** wobei der Behälter **11** ein metallisches Außengehäuse **11a** aufweist. Das metallische Außengehäuse besteht aus Stahl und weist eine Öffnung auf. Die Innenseite ist mit einer porösen bzw. absorbierenden Innenauskleidung **11c** versehen. Zwischen dem Außengehäuse und der Innenseite befindet sich ein Volumen, das evakuiert ist. Weiterhin ist ein Deckel **11d** zum Verschließen der Öffnung vorgesehen.

Weiterhin umfasst die netzunabhängige tragbare Vorrichtung **10** eine in das Innenvolumen **11e** des Behälters **11** einbringbare oder eine in dem Innenvolumen **11e** des Behälters **11** fest angeordnete Vorrichtung **1** für das Kühlen von biologischen und/oder medizinischen Proben.

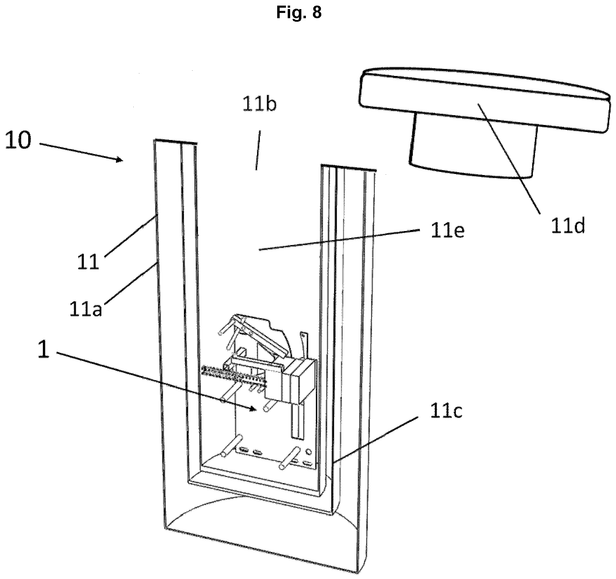

In Fig. 8 ist ein Schnitt durch eine schematische Perspektivansicht der Vorrichtung **10** für das Einfrieren und Kühlhalten von biologischen und/oder medizinischen Proben aus Fig. 7 gezeigt. Der Deckel **11d** zum Verschließen der Öffnung **11b** ist separat dargestellt.

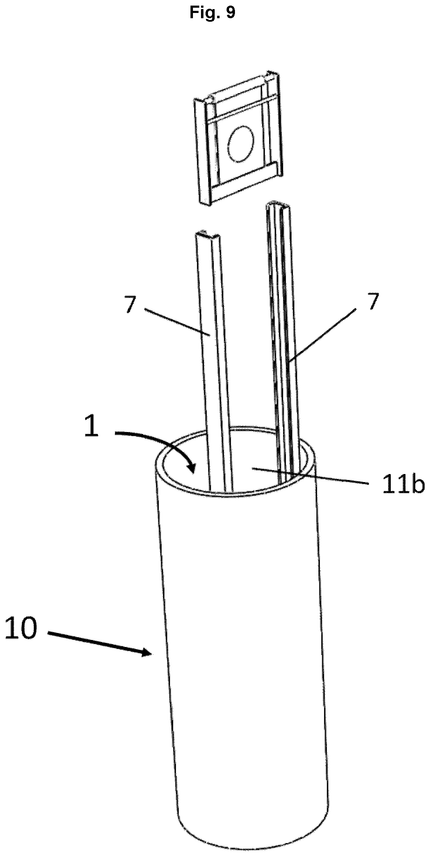

In Fig. 9 ist eine schematische Perspektivansicht der netzunabhängigen tragbaren Vorrichtung **10** für das Einfrieren und Kühlhalten gezeigt.

In dem Behälter der Vorrichtung **10** ist eine Vorrichtung **1** für das Kühlen von biologischen und/oder medizinischen Proben angeordnet. Eine Probe, die in einer Tüte in einer Probenkassette angeordnet ist, kann mittels der beiden Führungsvorrichtungen 7, die durch die Öffnung **11b** in den Behälter zu der Vorrichtung **1** für das Kühlen führen, zwischen die beiden Teiloberflächenelemente der Vorrichtung für das Kühlen gleitend geführt werden.

Es ist zu sehen, dass die Führungsvorrichtung **7** in deren oberem Bereich aus dem Behälter **10** herausragt. Nach dem Demontieren der Führungsvorrichtungen **7** ist der Behälter **10** verschließbar.

## Patentansprüche

1. Vorrichtung (1) für das Kühlen von biologischen und/oder medizinischen Proben (2), umfassend
> eine Einfriervorrichtung (3) für die biologischen und/oder medizinischen Proben (2),
> wobei die Einfriervorrichtung (3) aus vorkühlbaren Teiloberflächenelementen (3a, 3b) besteht,
> wobei die vorkühlbaren Teiloberflächenelemente (3a, 3b) aus einem Material mit einer geringen Kälteversprödung bestehen und eine Wärmeleitfähigkeit größer als 200 W/(m•K) aufweisen,
> wobei wenigstens eines der Teiloberflächenelemente (3a; 3b) gegenüber dem wenigstens einen weiteren Teiloberflächenelement (3b; 3a) beweglich angeordnet ist,
> wobei die Teiloberflächenelemente (3a, 3b) im zusammengefügten Zustand die Einfriervorrichtung (3) bilden und mit der zwischen den Teiloberflächenelementen befindlichen Probe (2) in thermisch wechselwirkendem Kontakt sind und
> wobei die Proben (2) im beabstandeten Zustand der Teiloberflächenelemente (3a, 3b) aus der Einfriervorrichtung (3) fallen, sowie
> eine Auslösevorrichtung (4),
➢ wobei die Auslösevorrichtung (4) einen gespannten Zustand aufweist sowie einen ausgelösten Zustand,
➢ wobei die Auslösevorrichtung (4) wenigstens ein Aktivierungsmittel aufweist, durch dessen Betätigung die Auslösevorrichtung (4) von dem gespannten Zustand in den ausgelösten Zustand bringbar ist,
➢ wobei die Auslösevorrichtung im gespannten Zustand wenigstens eines der Teiloberflächenelemente (3a; 3b) von dem wenigstens einen weiteren Teiloberflächenelement (3b; 3a) beabstandet hält und
➢ wobei im ausgelösten Zustand das wenigstens eine bewegliche Teiloberflächenelement (3a; 3b) und das wenigstens eine weitere Teiloberflächenelement (3b; 3a) aneinandergedrückt werden,
wobei die Vorrichtung mehrere Betriebszustände aufweist,
> wobei in einem ersten Betriebszustand der Vorrichtung zumindest die Einfriervorrichtung vorgekühlt ist, sowie die Auslösevorrichtung im gespannten Zustand ist und
> wobei in einem zweiten Betriebszustand der Vorrichtung zumindest die Einfriervorrichtung vorgekühlt ist, sowie die Auslösevorrichtung im ausgelösten Zustand ist.

2. Vorrichtung gemäß Anspruch 1, **dadurch gekennzeichnet, dass** die vorkühlbaren Teiloberflächenelemente (3a, 3b) aus Aluminium, Silber, Kupfer, Gold oder einer Kombination der vorgenannten Materialien bestehen.

3. Vorrichtung gemäß Anspruch 1 oder 2, **dadurch gekennzeichnet, dass** das wenigstens eine Aktivierungsmittel der Auslösevorrichtung (4) zur Überführung der Auslösevorrichtung von dem gespannten Zustand in den gelösten Zustand durch einen mechanischen und/oder magnetischen und/oder elektromagnetischen und/oder elektrischen und/oder optischen Auslöser (5) ausgestaltet wird.

4. Vorrichtung nach einem der Ansprüche 1 bis 3, **dadurch gekennzeichnet, dass** wenigstens ein Spannmittel vorhanden ist zur Überführung der Auslösevorrichtung (4) von dem ausgelösten Zustand in den gespannten Zustand durch einen mechanischen und/oder magnetischen und/oder elektromagnetischen und/oder elektrischen Spannmechanismus (6).

5. Vorrichtung nach einem der Ansprüche 1 bis 4, **dadurch gekennzeichnet, dass** der Vorrichtung (1) zum Kühlen wenigstens eine Führungsvorrichtung (7) zum gleitenden Einführen der Probe (2) in die Vorrichtung (1) zugeordnet ist.

6. Vorrichtung nach einem der Ansprüche 1 bis 5, **dadurch gekennzeichnet, dass** die biologische und/oder medizinische Probe (2) in einem Probenbehälter (8), insbesondere einer Tüte, einer Kanüle oder einem Röhrchen, anordbar ist.

7. Vorrichtung gemäß Anspruch 5 und Anspruch 6, **dadurch gekennzeichnet, dass** der Probenbehälter (8) in oder an einer Probenkassette (9) anordbar ist, wobei die Probenkassette (9) derart ausgestaltet ist, dass sie durch die wenigstens eine Führungsvorrichtung (7) führbar ist.

8. Vorrichtung nach Anspruch 3 oder einem der Ansprüche 4 bis 7 in Rückbeziehung auf Anspruch 3, **dadurch gekennzeichnet, dass** das wenigstens eine Aktivierungsmittel der Auslösevorrichtung (4) zur Überführung der Auslösevorrichtung von dem gespannten Zustand in den gelösten Zustand durch die Probenkassette (9) aktivierbar ist.

9. Vorrichtung nach den Ansprüchen 4 und 5 sowie nach den Ansprüchen 6 bis 8 in Rückbeziehung auf die Ansprüche 4 und 5, **dadurch gekennzeichnet, dass** das wenigstens eine Spannmittel der Auslösevorrichtung (4) zur Überführung der Auslösevorrichtung von dem ausgelösten Zustand in den gespannten Zustand durch einen mechanischen Spannmechanismus (6) realisiert ist, wobei das Mittel zum mechanischen Spannen des Spannmechanismus (6) zugleich die Führungsvorrichtung (7) zum gleitenden Einführen der Probe (2) in die Vorrichtung (1) ist.

10. Vorrichtung (10) für das Einfrieren und Kühlhalten von biologischen und/oder medizinischen Proben (2), umfassend
- einen Behälter (11) mit einem Außengehäuse (11a) mit einer Öffnung (11b) und mit einer porösen Innenauskleidung (11c) sowie einen Deckel (11d) zum Verschließen der Öffnung (11b) und
- eine in das Innenvolumen (11e) des Behälters (11) einbringbare oder eine in dem Innenvolumen des Behälters (11) festangeordnete Vorrichtung (1) für das Kühlen von biologischen und/oder medizinischen Proben gemäß einem der Ansprüche 1 bis 9.

11. Verfahren zum Einfrieren und Kühlhalten von biologischen und/oder medizinischen Proben (2) unter Verwendung einer zur Durchführung des folgenden Verfahrens vorbereiteten Vorrichtung (10) gemäß Anspruch 10, umfassend die Schritte:
a) Einbringen einer biologischen und/oder medizinischen Probe (2) in einen Probenbehälter (8),
b) Öffnen des Deckels (11d) zum Verschließen der Öffnung (11b) des Behälters (11),
c) Spannen der Auslösevorrichtung (4) der Vorrichtung zum Kühlen (1),
d) Einbringen des mit der biologischen und/oder medizinischen Probe (2) versehenen Probenbehälters (8) zwischen die Teiloberflächenelemente (3a, 3b) der Einfriervorrichtung (3),
e) Überführen der Auslösevorrichtung (4) vom gespannten Zustand in den ausgelösten Zustand,
f) Warten,
g) Überführen der Auslösevorrichtung (4) vom ausgelösten Zustand in den gespannten Zustand und/oder
h) Einsetzen des Deckels (11d) zum Verschließen der Öffnung (11b) des Behälters (11).

12. Verfahren zum Einfrieren und Kühlhalten von biologischen und/oder medizinischen Proben (2) unter Verwendung einer zur Durchführung des folgenden Verfahrens vorbereiteten Vorrichtung (10) gemäß Anspruch 10, umfassend die Schritte:
a) Einbringen einer biologischen und/oder medizinischen Probe (2) in einen Probenbehälter (8),
b) Öffnen des Deckels (11d) zum Verschließen der Öffnung (11b) des Behälters (11),
c) Spannen der Auslösevorrichtung (4) der Vorrichtung zum Kühlen (1),
d) Einbringen des mit der biologischen und/oder medizinischen Probe (2) versehenen Probenbehälters (8) zwischen die Teiloberflächenelemente (3a, 3b) der Einfriervorrichtung (3) und dadurch bedingtes Überführen der Auslösevorrichtung (4) vom gespannten Zustand in den ausgelösten Zustand,
e) Warten
f) Überführen der Auslösevorrichtung (4) vom ausgelösten Zustand in den gespannten Zustand und/oder
g) Einsetzen des Deckels (11d) zum Verschließen der Öffnung (11b) des Behälters (11).

## Claims

1. Device (1) for the cooling of biological and/or medical samples (2), comprising
➢ a freezing device (3) for the biological and/or medical samples (2),
➢ wherein the freezing device (3) consists of precoolable surface subelements (3a, 3b),
➢ wherein the precoolable surface subelements (3a, 3b) consist of a material having low cold brittleness and a thermal conductivity greater than 200 W/(m•K),
➢ wherein at least one of the surface subelements (3a; 3b) is arranged so as to be movable relative to the at least one further surface subelement (3b; 3a),
➢ wherein the surface subelements (3a, 3b), when fitted together, form the freezing device (3) and are in thermally interacting contact with the sample (2) positioned between the surface subelements and
➢ wherein the samples (2) drop out of the freezing device (3) when the surface subelements (3a, 3b) are spaced apart, and also
➢ a release device (4),
➢ wherein the release device (4) has a tensioned state and a released state,
➢ wherein the release device (4) comprises at least one activation means, the actuation of which can bring the release device (4) from the tensioned state into the released state,
➢ wherein the release device holds at least one of the surface subelements (3a; 3b) apart from the at least one further surface subelement (3b; 3a) in the tensioned state and
➢ wherein the at least one movable surface subelement (3a; 3b) and the at least one further surface subelement (3b; 3a) are pressed against one another in the released state,
wherein the device comprises a number of operating states,
➢ wherein in a first operating state of the device, at least the freezing device is precooled and the release device is in the tensioned state, and
➢ wherein in a second operating state of the device, at least the freezing device is precooled and the release device is in the released state.

2. Device according to claim 1, **characterised in that** the precoolable surface subelements (3a, 3b) consist of aluminium, silver, copper, gold or a combination of said materials.

3. Device according to claim 1 or 2, **characterised in that** the at least one activation means of the release device (4) is designed to switch the release device from the tensioned state into the released state by means of a mechanical and/or magnetic and/or electromagnetic and/or electric and/or optical trigger (5).

4. Device according to one of claims 1 to 3, **characterised in that** at least one tensioning means is present for switching the release device (4) from the released state into the tensioned state by means of a mechanical and/or magnetic and/or electromagnetic and/or electric tensioning mechanism (6).

5. Device according to one of claims 1 to 4, **characterised in that** at least one guidance device (7) for slidingly introducing the sample (2) into the device (1) is associated with the cooling device (1).

6. Device according to one of claims 1 to 5, **characterised in that** the biological and/or medical sample (2) can be arranged in a sample container (8), in particular a bag, a cannula or a vial.

7. Device according to claim 5 and claim 6, **characterised in that** the sample container (8) can be arranged in or on a sample cartridge (9), wherein the sample cartridge (9) is designed in such a way that it can be guided by the at least one guidance device (7).

8. Device according to claim 3 or to one of claims 4 to 7 referring back to claim 3, **characterised in that** the at least one activation means of the release device (4) for switching the release device from the tensioned state into the released state can be activated by the sample cartridge (9).

9. Device according to claims 4 and 5 and according to claims 6 to 8 referring back to claims 4 and 5, **characterised in that** the at least one tensioning means of the release device (4) for switching the release device from the released state into the tensioned state is in the form of a mechanical tensioning mechanism (6), wherein the means for the mechanical tensioning of the tensioning mechanism (6) is also the guidance device (7) for slidingly introducing the sample (2) into the device (1).

10. Device (10) for the freezing and keeping cool of biological and/or medical samples (2), comprising
- a container (11) having an outer casing (11a) with an opening (11b) and having a porous inner lining (11c) as well as a cover (11d) for closing the opening (11b) and
- a device (1) that can be introduced into the interior (11e) of the container (11) or that is fixedly arranged in the interior of the container (11) for the cooling of biological and/or medical samples according to one of claims 1 to 9.

11. Method for freezing and keeping cool biological and/or medical samples (2), using a device (10) according to claim 10 provided for performing the following method, comprising the following steps:
a) introducing a biological and/or medical sample (2) into a sample container (8),
b) opening the cover (11d) for closing the opening (11b) of the container (11),
c) tensioning the release device (4) of the cooling device (1),
d) introducing the sample container (8) furnished with the biological and/or medical sample (2) between the surface subelements (3a, 3b) of the freezing device (3),
e) switching the release device (4) from the tensioned state into the released state,
f) waiting,
g) switching the release device (4) from the released state into the tensioned state, and/or
h) inserting the cover (11d) for closing the opening (11b) of the container (11).

12. Method for freezing and keeping cool biological and/or medical samples (2), using a device (10) according to claim 10 provided for performing the following method, comprising the following steps:
a) introducing a biological and/or medical sample (2) into a sample container (8),
b) opening the cover (11d) for closing the opening (11b) of the container (11),
c) tensioning the release device (4) of the cooling device (1),
d) introducing the sample container (8) furnished with the biological and/or medical sample (2) between the surface subelements (3a, 3b) of the freezing device (3), thereby switching the release device (4) from the tensioned state into the released state,
e) waiting,
f) switching the release device (4) from the released state into the tensioned state, and/or
g) inserting the cover (11d) for closing the opening (11b) of the container (11).

## Revendications

1. Dispositif (1) de refroidissement d'échantillons biologiques et/ou médicaux (2), comprenant
- un dispositif de congélation (3) pour les échantillons biologiques et/ou médicaux (2),
- le dispositif de congélation (3) étant constitué d'éléments de surface partiels (3a, 3b) pouvant être prérefroidis,
- les éléments de surface partiels pouvant être prérefroidis (3a, 3b) étant constitués d'un matériau présentant une faible fragilité au froid et ayant une conductivité thermique supérieure à 200 W/(m·K),
- au moins l'un des éléments de surface partiels (3a ; 3b) étant disposé mobile par rapport à au moins un autre élément de surface partiel (3b ; 3a),
- les éléments de surface partiels (3a, 3b) formant, à l'état réuni, le dispositif de congélation (3) et étant en contact d'interaction thermique avec l'échantillon (2) situé entre les éléments de surface partiels, et
- les échantillons (2) tombant du dispositif de congélation (3) à l'état espacé des éléments de surface partiels (3a, 3b), et
- un dispositif de déclenchement (4),
- le dispositif de déclenchement (4) présentant un état armé ainsi qu'un état désarmé,
- le dispositif de déclenchement (4) présentant au moins un moyen d'activation, dont l'actionnement peut faire passer le dispositif de déclenchement (4) de l'état armé à l'état désarmé,
- le dispositif de déclenchement maintenant, à l'état armé, au moins l'un des éléments de surface partiels (3a ; 3b) à distance dudit au moins un autre élément de surface partiel (3b ; 3a), et
- ledit au moins un élément de surface partiel mobile (3a ; 3b) et ledit au moins un autre élément de surface partiel (3b ; 3a) étant pressés l'un contre l'autre à l'état désarmé,
le dispositif présentant plusieurs états de fonctionnement,
- dans un premier état de fonctionnement du dispositif, au moins le dispositif de congélation étant prérefroidi, et le dispositif de déclenchement étant à l'état armé, et
- dans un deuxième état de fonctionnement du dispositif, au moins le dispositif de congélation étant prérefroidi, et le dispositif de déclenchement étant à l'état désarmé.

2. Dispositif selon la revendication 1, **caractérisé en ce que** les éléments de surface partiels (3a, 3b) pouvant être prérefroidis sont en aluminium, en argent, en cuivre, en or ou en une combinaison des matériaux précités.

3. Dispositif selon la revendication 1 ou 2, **caractérisé en ce que** ledit au moins un moyen d'activation du dispositif de déclenchement (4) est configuré pour faire passer le dispositif de déclenchement de l'état armé à l'état désarmé par le biais d'un déclencheur (5) mécanique et/ou magnétique et/ou électromagnétique et/ou électrique et/ou optique.

4. Dispositif selon l'une des revendications 1 à 3, **caractérisé en ce qu'il** est prévu au moins un moyen d'armement pour faire passer le dispositif de déclenchement (4) de l'état désarmé à l'état armé par le biais d'un mécanisme d'armement (6) mécanique et/ou magnétique et/ou électromagnétique et/ou électrique.

5. Dispositif selon l'une des revendications 1 à 4, **caractérisé en ce qu'au** moins un dispositif de guidage (7) est associé au dispositif (1) de refroidissement pour l'introduction par glissement de l'échantillon (2) dans le dispositif (1).

6. Dispositif selon l'une des revendications 1 à 5, **caractérisé en ce que** l'échantillon biologique et/ou médical (2) peut être disposé dans un récipient d'échantillon (8), notamment un sachet, une canule ou un tube.

7. Dispositif selon la revendication 5 et la revendication 6, **caractérisé en ce que** le récipient d'échantillon (8) peut être disposé dans ou sur une cassette d'échantillon (9), la cassette d'échantillon (9) étant conçue de telle sorte qu'elle peut être guidée par ledit au moins un dispositif de guidage (7).

8. Dispositif selon la revendication 3 ou l'une des revendications 4 à 7 associée à la revendication 3, **caractérisé en ce que** ledit au moins un moyen d'activation du dispositif de déclenchement (4) peut être activé par la cassette d'échantillons (9) pour faire passer le dispositif de déclenchement de l'état armé à l'état désarmé.

9. Dispositif selon les revendications 4 et 5 et selon les revendications 6 à 8 associées aux revendications 4 et 5, **caractérisé en ce que** ledit au moins un moyen d'armement du dispositif de déclenchement (4) pour faire passer le dispositif de déclenchement de l'état désarmé à l'état armé est réalisé sous la forme d'un mécanisme d'armement mécanique (6), le moyen d'armement mécanique du mécanisme d'armement (6) étant également le dispositif de guidage (7) pour l'introduction par glissement de l'échantillon (2) dans le dispositif (1).

10. Dispositif (10) de congélation et de maintien au froid d'échantillons biologiques et/ou médicaux (2), comprenant
- un récipient (11) muni d'un boîtier extérieur (11a) avec une ouverture (11b) d'un revêtement intérieur poreux (11c) ainsi qu'un couvercle (11d) pour fermer l'ouverture (11b) et
- un dispositif (1) de refroidissement d'échantillons biologiques et/ou médicaux selon l'une des revendications 1 à 9, pouvant être inséré dans le volume interne (11e) du récipient (11) ou disposé de façon fixe dans le volume interne du récipient (11).

11. Procédé de congélation et de maintien au froid d'échantillons biologiques et/ou médicaux (2) en utilisant un dispositif (10) selon la revendication 10préparé pour la mise en œuvre du procédé suivant, comprenant les étapes consistant à :
a) Introduire un échantillon biologique et/ou médical (2) dans un récipient d'échantillon (8),
b) Ouvrir le couvercle (11d) destiné à fermer l'ouverture (11b) du récipient (11),
c) Armer le dispositif de déclenchement (4) du dispositif de refroidissement (1),
d) Introduire le récipient d'échantillon (8) muni de l'échantillon biologique et/ou médical (2) entre les éléments de surface partiels (3a, 3b) du dispositif de congélation (3),
e) Faire passer le dispositif de déclenchement (4) de l'état armé à l'état désarmé,
f) Attendre,
g) Faire passer le dispositif de déclenchement (4) de l'état désarmé à l'état armé et/ou
h) Mettre en place le couvercle (11d) pour fermer l'ouverture (11b) du récipient (11).

12. Procédé de congélation et de maintien au froid d'échantillons biologiques et/ou médicaux (2) en utilisant un dispositif (10) selon la revendication 10 préparé pour la mise en œuvre du procédé suivant, comprenant les étapes consistant à :
a) Introduire un échantillon biologique et/ou médical (2) dans un récipient d'échantillon (8),
b) Ouvrir le couvercle (11d) destiné à fermer l'ouverture (11b) du récipient (11),
c) Armer le dispositif de déclenchement (4) du dispositif de refroidissement (1),
d) Introduire le récipient d'échantillon (8) muni de l'échantillon biologique et/ou médical (2) entre les éléments de surface partiels (3a, 3b) du dispositif de congélation (3) et, de ce fait, faire passer le dispositif de déclenchement (4) de l'état armé à l'état désarmé,
e) Attendre
f) Faire passer le dispositif de déclenchement (4) de l'état désarmé à l'état armé et/ou
g) Mettre en place le couvercle (11d) pour fermer l'ouverture (11b) du récipient (11).
